(19) Europäisches Patentamt
European Patent Office
Office européen des brevets

(11) Veröffentlichungsnummer : **0 124 766 B1**

(12) # EUROPÄISCHE PATENTSCHRIFT

(45) Veröffentlichungstag der Patentschrift : **09.08.89**

(51) Int. Cl.$^4$ : **A 61 K 49/00**

(21) Anmeldenummer : **84103699.9**

(22) Anmeldetag : **04.04.84**

(54) **Enterales Kontrastmittel für die Kernspintomographie und dessen Herstellung.**

(30) Priorität : **04.05.83 DE 3316703**

(43) Veröffentlichungstag der Anmeldung : **14.11.84 Patentblatt 84/46**

(45) Bekanntmachung des Hinweises auf die Patenterteilung : **09.08.89 Patentblatt 89/32**

(84) Benannte Vertragsstaaten : **AT BE CH DE FR GB IT LI LU NL SE**

(56) Entgegenhaltungen :
**EP–A– 0 071 564**
**BIOLOGICAL ABSTRACTS, Band 78, Nr. 3, 1984, Seite 2515, Zusammenfassung Nr. 22094; R. GROSSMAN et al.: "Gadolinium enhanced NMR images of experimental brain abscess", & J. COMPUT. ASSIST. TOMOGR. 8(2): 204-207, 1984**
**CHEMICAL ABSTRACTS, Band 99, Nr. 7, 15. August 1983, Seite 232, Zusammenfassung Nr. 49843k, Columbus, Ohio, US; V.M. RUNGE et al.: "Work in progress: potential oral and intravenous paramagnetic NMR contrast agents", & RADIOLOGY (EASTON PA.) 1983, 147(3), 789-91**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Weinmann, Hanns-Joachim, Dr.**
**Westhofener Weg 27**
**D-1000 Berlin 38 (DE)**
Erfinder : **Gries, Heinz, Dr.**
**Helmstedter Strasse 19**
**D-1000 Berlin 31 (DE)**
Erfinder : **Michel, Heinrich, Dr.**
**Seeburger Strasse 5**
**D-1000 Berlin 20 (DE)**

**EP 0 124 766 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue enterale Kontrastmittel für die Protonenkernspintomographie gemäß der Ansprüche 1 bis 12.

Die Kernspintomographie hat sich zu einer neuen und sehr leistungsfähigen bildgebenden diagnostischen Methode entwickelt, die den bekannten Diagnoseverfahren (Röntgendiagnostik, Röntgencomputertomographie) bei der Lösung bestimmter diagnostischer Probleme überlegen ist.

So beruht ein Vorteil der Kernspintomographie auf ihrer hervorragenden Eignung zur Differenzierung von Weichteilgeweben. Durch die Verwendung von Kontrastmitteln bei der Kernspintomographie wird der Informationsgehalt der Aufnahmen bedeutend erhöht. Hierbei finden neuartige jodfreie Kontrastmittel in teilweise sehr niedrigen Dosierungen Verwendung, die wesentlich besser verträglich sind als jodhaltige Kontrastmittel. Die für die Kernspintomographie geeigneten Kontrastmittel haben durch ihren Paramagnetismus die Eigenschaft, die Relaxationszeiten $T_1$ und $T_2$ der im Körperwasser vorhandenen Wasserstoffatome so zu beeinflussen, daß die Bildgebung während der Aufnahme mit einem Kernspintomographen wesentlich verbessert wird.

Da aber die Möglichkeit der Bildgewinnung von Geweben durch die Kernspintomographie nur besteht, wenn genügend Wasserstoffatome (Wasser, Fett) vorhanden sind, lassen sich Körperhöhlen nur dann durch die Kernspintomographie darstellen, wenn sie mit einer ausreichenden Menge Wasser bzw. Fett gefüllt sind und diese flüssigkeitsmenge während der Aufnahmezeit in diesem zu diagnostizierenden Areal gehalten werden kann.

Erst dann kann auch das paramagnetische Kontrastmittel seine Wirkung entfalten.

Die bisher beschriebenen Kontrastmittel für die Kernspintomographie (DE-A-31 29 906 und DE-A-34 01 052) sind nur für die Darstellung von Geweben mit ausreichendem Wasser- oder Fettgehalt geeignet.

Gemäß der vorliegenden Erfindung gelingt eine länger anhaltende durch das paramagnetische Kontrastmittel verstärkte Bildgebung des Magen-Darm-Kanals dadurch, daß man eine wäßrige paramagnetische Kontrastmittellösung, die neben Base/Puffer oder Puffersubstanz mit einem pH-Wert von 3 bis 8 ein osmotisch wirksames Polyol enthält, oral bzw. rektal verabreicht.

Durch die gleichzeitige Gabe dieses osmotisch wirksamen Polyols erzeugt man im Magen-Darm-Kanal eine zur übrigen Körperflüssigkeit iso- bzw. hypertone Lösung, durch die ein ausreichend hoher osmotischer Druck der Kontrastmittellösung sichergestellt und die Resorption des für die Bildgebung erforderlichen Wassers verhindert bzw. stark verlangsamt wird.

Ist der osmotisch wirksame Zusatz nicht vorhanden, wird das mit der Kontrastmittel-Lösung verabreichte Wasser rasch resorbiert, wodurch die für die Signalgebung erforderliche Menge an Wasserstoffatomen schnell abnimmt. Das führt zu einer Konzentrationserhöhung der paramagnetischen Verbindung und diese wiederum zur Abnahme oder zum völligen Verlust der Signalintensität während der Passage des Kontrastmittels durch den Darmtrakt.

Die paramagnetischen Kontrastmittel für die Kernspintomographie des Standes der Technik sind deshalb bei oraler Anwendung nur für die Kontrastierung des Magens geeignet.

Mit den in der Literatur beschriebenen Kontrastmittellösungen ist deshalb nach oraler Verabreichung eine nur kurze Darstellung des Magen-Darm-Kanals möglich, da das mit der Kontrastmittel-Lösung aufgenommene Wasser vom Gastrointestinalkanal rasch resorbiert wird.

Es war daher wünschenswert, ein paramagnetisches Kontrastmittel für die Kernspintomographie zur Verfügung zu stellen, das eine länger anhaltende Kontrastierung des Magen-Darm-Kanals ermöglicht.

Gemäß der Erfindung sind zur Herstellung des Kontrastmittels alle physiologisch verträglichen paramagnetischen Verbindungen geeignet, insbesondere

Eisen-III-Verbindungen wie
Eisen-III-ammoniumcitrat,
Eisen-III-glycerophosphat oder
Eisen-III-sulfat sowie

die paramagnetischen Komplexsalze, wie sie beispielsweise in der Europäischen Patentanmeldung EP-A-71564 und in der DE-A-34 01 052.1 beschrieben sind. Diese Komplexsalze bestehen aus dem Anion einer komplexbildenden Säure und einem oder mehreren Zentralionen und gegebenenfalls einem oder mehreren physiologisch unbedenklichen Kationen einer anorganischen und/oder organischen Base oder Aminosäure.

Da das erfindungsgemäße Mittel zur Anwendung in der NMR-Diagnostik bestimmt ist, muß das Zentralion des Komplexsalzes paramagnetisch sein. Dies sind insbesondere die zweiund dreiwertigen Ionen der Elemente der Ordnungszahlen 21 bis 29, 42, 44 und 58 bis 70. Geeignete Ionen sind beispielsweise das Chrom(III)-, Mangan(II)-, Eisen(III)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)- und Erbium(III)-ion.

Als komplexbildende Säuren sind solche geeignet, die man üblicherweise zur Komplexbildung der obengenannten Zentralionen verwendet. Geeignete komplexbildende Säuren sind beispielsweise solche,

die 3 bis 12, vorzugsweise 3 bis 8 Methylenphosphonsäure-Gruppen, Methylencarbohydroxamsäure-Gruppen, Carboxyethylidengruppen oder insbesondere Carboxymethylengruppen enthalten, von denen jeweils eine oder zwei an ein die Komplexbildung unterstützendes Stickstoffatom gebunden sind. Der Stickstoff ist über ein gegebenenfalls substituiertes Ethylen oder über bis zu vier, jeweils durch ein die Komplexbildung unterstützendes Stickstoff- oder Sauerstoff- oder Schwefelatom getrennte Ethyleneinheiten an ein weiteres Stickstoffatom gebunden.

Geeignete Komplexsalze sind beispielsweise solche der allgemeinen Formel Ia

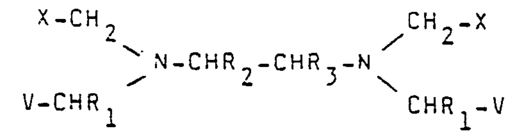

(Ia)

worin

X die Reste —COOY, —$PO_3$HY oder —CONHOY mit Y in der Bedeutung eines Wasserstoffatoms, eines Metallionenäquivalentes und/oder eines physiologisch unbedenklichen Kations einer anorganischen oder organischen Base oder Aminosäure bedeutet und worin $R_1$ Wasserstoffatome oder Methylgruppen, $R_2$ und $R_3$ gemeinsam eine Trimethylengruppe oder eine Tetramethylengruppe oder Wasserstoffatome, niedere Alkylgruppen, Phenylgruppen, Benzylgruppen darstellen und worin

V die gleiche Bedeutung wie X besitzt, oder die Gruppen —$CH_2$OH oder —CONH$(CH_2)_n$X mit X in der obenangegebenen Bedeutung und n in der Bedeutung der Ziffern 1 bis 12, darstellt, mit der Maßgabe, das mindestens zwei der Substituenten Y Metallionenäquivalente eines Elements der Ordnungszahlen 21 bis 29, 42, 44 oder 58 bis 70 darstellen.

Zur Herstellung der Komplexsalze der allgemeinen formel Ia eignen sich unter anderem folgende komplexbildende säuren: die Ethylendiamintetraessigsäure, die Ethylendiamintetraacethydroxamsäure, die trans-1.2-Cyclohexylendiamintetraessigsäure, die DL-2.3-Butylendiamintetraessigsäure, die DL-1.2-Butylendiamintetraessigsäure, die DL-1.2-Propylendiamintetraessigsäure, die 1.2-Diphenylethylendiamintetraessigsäure, die Ethylendinitrolotetrakis (methanphosphonsäure) und die N-(2-Hydroxyethyl)-ethylendiamin-triessigsäure.

Weitere geeignete Komplexsalze sind beispielsweise solche der allgemeinen Formel Ib

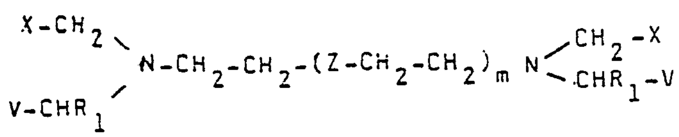

(Ib)

worin X, V und $R_1$ die obengenannte Bedeutung besitzen, m für die Zahlen 1, 2 oder 3 steht und Z ein Sauerstoffatom oder ein schwefelatom oder die Gruppe

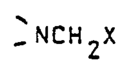 oder 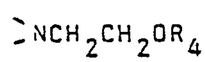

mit X in der obenangegebenen Bedeutung und $R_4$ in der Bedeutung einer niederen Alkylgruppe darstellt. Falls Z ein Sauerstoffatom oder ein Schwefelatom bedeutet, sind Komplexsalze mit m in der Bedeutung von 1 oder 2 bevorzugt. Zur Herstellung der Komplexsalze der allgemeinen Formel Ib eignen sich unter anderem folgende komplexbildenden Säuren: die Diethylentriaminpentaessigsäure, die Triethylentetraminhexaessigsäure, die Tetraethylenpentaminheptaessigsäure, die 13.23-Dioxo-15.18.21-tris(carboxymethyl)-12.15.18.21.24-pentaazapentatriacontandisäure, die 3.9-Bis-(1-carboxyethyl)-3.6.9-triazaundecandisäure, die Diethylentriaminpentakis-(methylenphosphonsäure), die 1.10-Diaza-4.7-dioxadecan-1.1.-10.10-tetraessigsäure und die 1.10-Diaza-4.7-dithiadecan-1.1.10.10-tetraessigsäure.

Geeignete Komplexsalze sind ferner solche der allgemeinen Formel Ic

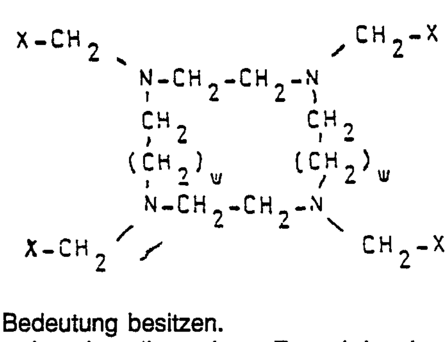

(Ic)

worin X und w die obengenannte Bedeutung besitzen.

Zur Herstellung der Komplexsalze der allgemeinen Formel Ic eignen sich unter anderem folgende komplexbildende Säuren: die 1.4.8.11-Tetraazacyclotetradecantetraessigsäure und insbesonders die 1.4.7.10-Tetraazacyclododecantetraessigsäure.

Wenn nicht alle aziden Wasserstoffatome der komplexbildenden Säure durch des Zentralion oder die Zentralionen substituiert werden, ist es zwecks Erhöhung der Löslichkeit des Komplexsalzes zweckmäßig, die verbleibenden Wasserstoffatome durch physiologisch unbedenkliche Kationen anorganischer und-/oder organischer Basen oder Aminosäuren zu substituieren. Geeignete anorganische Kationen sind beispielweise das Lithiumion, das Kaliumion oder insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N, N-Dimethylglucamin oder insbesondere N-Methylglucamin. Geeignete Kationen von Aminosäuren sind beispielsweise die des Lysins, Arginins oder Ornithins.

Die für das erfindungsgemäße Mittel benötigten komplexbildenden Säuren sind bekannt, oder können in an sich bekannter Weise hergestellt werden.

Besonders hervorgehoben seien das Di-N-Methylglucaminsalz des Gadolinium-III-Komplexes der Dietylentriminpentaessigsäure (DTPA), das Di-Natriumsalz des Gadolinium-III-Komplexes der DTPA, das N-Methylglucain-Natrium-Mischsalz der DTPA, das Di-N-Methylglucainsalz des Eisen-III-Komplexes der DTPA und das Natriumsalz des Eisen-III-Komplexes der Äthylendiamintetraessigsäure (EDTA), wobei das Di-N-Methylglucaminsalz des Gadolinium-III-Komplexes der DTPA bevorzugt ist.

Soweit in der Literatur nicht beschrieben, können die physiologisch verträglichen paramagnetischen Komplexsalze nach dem Fachmann bekannten Verfahren hergestellt werden, wie das folgende Herstellungsbeispiel zeigen soll. Da sich die Mono-N-methylglucaminsalze besser isolieren und handhaben lassen, stellt man zunächst diese her und gibt bei der Verwendung dieser Mono-Salze noch ein weiteres Äquivalent N-Methylglucamin zu :

Herstellung von Mono-N-methylglucaminsalz des Eisen-III-Komplexes der Diethylentriaminpentaessigsäure

23,6 g (60 mMol) Diethylentriamin-pentaessigsäure werden in einer Lösung von 16,6 g (60 mMol) Eisen-III-Chlorid-Hexahydrat ($FeCl_3 \cdot 6H_2O$, 98 %ig) in 500 ml Wasser suspendiert. Dann setzt man tropfenweise unter intensivem Rühren 180 ml einer n-Natronlauge (180 mMol) zu, wobei sich ein pH-Wert von 3,5 einstellt. Dann erhitzt man den Ansatz zwei Stunden auf 95 °C, saugt den ausgeschiedenen gelben Niederschlag ab und wäscht ihn mit Wasser. Der feuchte Niederschlag wird in 200 ml Wasser suspendiert und nach Zugabe von 1,95 g (10 mMol) N-Methylglucamin durch vierstündiges Erhitzen auf 95 °C in Lösung gebracht. Die klare rotbraune Lösung wird anschließend im Vakuum zur Trockne eingeengt. Nach Trocknen bei 60 °C im Vakuum erhält man in quantitativer Ausbeute ein braunes Pulver vom Schmelzpunkt 131-133 °C (Aufschäumen).

Analyse ($C_{21}H_{36}FeN_4O_{15}$, M = 641) :

| | | | | |
|---|---|---|---|---|
| Berechnet : | C 39,88 % | H 5,74 % | N 8,86 % | Fe 8,83 % |
| Gefunden : | C 39,77 % | H 5,90 % | N 8,68 % | Fe 8,80 %. |

Die paramagnetischen Komplexsalze werden in einer Konzentration von 0,05 bis 500 mMol/l, vorzugsweise 0,5 bis 20 mMol/l, verwendet.

Als physiologisch verträgliche osmotisch wirksame Substanzen werden Polyole wie beispielsweise Mannit, Sorbit, Arabit und Xylit verwendet, wobei Mannit und Sorbit bevorzugt sind.

Die Konzentration der osmotisch wirksamen Substanz beträgt 5 bis 70 g pro Liter, vorzugsweise 30 bis 50 g pro Liter.

Zur Stabilisierung der paramagnetische Komplexsalze gegenüber dem sauren Mageninhalt ist der Zusatz von Substanzen erforderlich, die die Wasserstoffionen-Konzentration des Mageninhaltes so erniedrigen, daß der pH-Wert größer als 3 ist. Zu diesem Zweck verwendet man Basen oder Puffer bzw. Puffergemische, die diesen pH-Bereich gewährleisten und physiologisch verträglich sind wie beispielsweise Trishydroxymethylaminomethan (2-Amino-2-hydroxymethyl-1,3-propandiol, Tromethamol), Natriumdihydrogenphosphat/Dinatriumhydrogenphosphat oder Zitronensäure/Dinatriumphosphat, wobei Tromethamol bevorzugt ist.

Diese Substanzen werden in einer Menge von 10 bis 50 mMol pro Liter verwendet.

Zur besseren Füllung des Magen-Darm-Kanals und zur Verfestigung des Stuhles kann es erforderlich sein, dem erfindungsgemäßen Mittel gegebenfalls Stoffe zuzusetzen, die die Viskosität des Kontrastmittels erhöhen und physiologisch unbedenklich sind. Die dafür infrage kommenden viskositätserhöhend wirkenden Substanzen sind natürliche, hochmolekulare Kohlenhydrate wie Alginate, Xanthan-Gummi, Pektin, Traganth, Bassorin, Guar, Karaya, Gummi arbicum oder Polypeptide wie Casein und Gelatine oder halbsynthetische hochmolekulare Kohlenhydrate wie mikrokristalline Cellulose, Natriumcarboxymethylcellulose, Methylcellulose und deren Hydroxyalkylderivate wie Methylhydroxyethylcellulose oder quellfähige Silikate wie Bentonit und kolloidale Kieselsäure oder die als Antidiarrhoika verwendeten Präparate.

Die viskositätserhöhenden Stoffe verwendet man gegebenenfalls in einer Menge von 2 bis 40 Gramm pro Liter, vorzugsweise von 10 bis 30 Gramm pro Liter.

Die erfindungsgemäßen Kontrastmittel können zur Untersuchung bestimmter Läsionen des Enddarmes wie Rektum oder Kolon auch rektal angewendet werden, wobei zweckmäßigerweise das Kontrastmittel in den entleerten Darm gegeben wird.

Die Erfindung betrifft auch ein Verfahren zur Herstellung der neuen enteralen Kontrastmittel für die Kernspintomographie gemäß Anspruch 12.

Zur Herstellung der neuen Mittel werden 65 g bis 70 g des kontrastmittelhaltigen Granulates mit 1 Liter Wasser vermischt und die erhaltene Suspension bzw. Lösung sofort nach der Herstellung bis spätestens 5 Minuten danach in den Magen-Darm-Kanal gebracht, was durch Verschlucken oder mittels einer Schlundsonde geschieht.

Man benötigt 2 ml bis 30 ml Kontrastmittel pro Kilogramm Körpergewicht.

Das für die Herstellung des Kontrastmittel gemäß Anspruch 14 benötigte Granulat erhält man, indem man die osmotisch wirksame Substanz gegebenenfalls mit dem viskositätserhöhenden Stoff und falls erwünscht, mit Gesmackskorrigentien vermischt und durch ein Sieb gibt und — für den Fall, daß der viskositätserhöhende Stoff vorhanden ist — nochmals mischt. Man erhält ein Pulver, das mit soviel einer Granulierlösung portionsweise befeuchtet und angerieben wird, daß das entstehende feuchte Granulat anschließend nochmals gesiebt werden kann.

Die Granulierlösung erhält man, indem das paramagnetische Komplexsalz mit der Puffersubstanz und Meglumin in Wasser löst, mit anorganischer verdünnter Säure auf den pH-Wert 7,4 bis 7,5 einstellt und auf das gewünschte Volumen mit Wasser auffüllt.

Nachdem mit dieser Granulierlösung — wie oben beschrieben das erhaltene Pulver befeuchtet und angerieben wurde, gibt man dieses wiederum durch ein Sieb, trocknet das Siebgut bie 50 °C und vermindertem Druck (200 Torr) für 1,5 bis 3 Stunden, egalisiert das getrocknete Granulat durch nochmaliges Sieben und mischt abschließend ein letztes Mal.

Man erhält so 65 bis 70 g Granulat das durch Auflösen in 1 Liter Wasser das gebrauchsfertige Arzneimittel liefert.

Die nachfolgende Arbeitsvorschrift soll die Anwendung der erfindungsgemäßen neuen Kontrastmittel näher erläutern :

Einer männlichen, etwa 400 g schweren Sprague-Dawley Ratte werden mit Hilfe einer Sonde 5 ml eines Kontrastmittels folgender Zusammensetzung verabreicht :

| | |
|---|---|
| 1 mMol/l | Gadoliniumkomplex der Diäthylentriaminpentaessigsäure als Di-N-Methylglucaminsalz |
| 20 mMol/l | Trometham ol |
| 45 g/l | Mannit. |

Die Lösung war mit 1n Salzsäure auf pH = 7,2 eingestellt.

Nach der Applikation wird das Tier durch eine intraperitoneale Injektion von Pentobarbital-Natrium (60 mg/kg) narkotisiert. Anschließend werden horizontale Schichten in Höhe des Abdomens der Ratte mit Hilfe eines Kleintier-Protonenkernspin-Tomographen durchgeführt. In den ersten etwa 10 Minuten nach oraler Gabe aufgenommenen Aufnahmen ist der Mageninhalt weiß kontrastiert und sehr gut von den parenchymatösen Organen des Abdomens abzugrenzen. In späteren Aufnahmen können einwandfrei Darmschlingen dargestellt werden. Dieses ist nur durch den Zusatz des nicht-resorbierbaren Mannit möglich. Ohne diesen osmotisch wirksamen Zusatz wird das Wasser zu schnell resorbiert, und eine Kontrastierung des Darmlumens ist nicht möglich.

## Herstellungsbeispiel 1

A) Herstellung der Granulierlösung

Man löst 743 mg (1 mMol) Mono-N-Methylglucaminsalz des Gadolinium-III-Komplexes der Diäthylentriaminpentaessigsäure (DTPA) mit 195,2 mg (1 mMol) N-Methylglucamin und 2,423 g (20 mMol) Trishydroxymethylaminomethan in 5 ml Wasser, stellt mit 1n Salzsäure den pH-Wert auf 7,4 bis 7,5 ein und füllt mit Wasser auf ein Volumen von 10 ml auf.

B) Man mischt 45 g Mannit und 20 g Methylhydroxyethylcellulose ca. 3 Minuten, gibt die Mischung durch ein Sieb mit 0,8 mm Maschenweite und mischt nochmals 3 Minuten. Danach wird das erhaltene Pulver mit der nach A) hergestellten Granulierlösung (10 ml) portionsweise befeuchtet und angerieben, das feuchte Granulat durch ein Sieb mit 1,25 mm Maschenweite gegeben, danach 2 Stunden bei 50 °C und 27 kPa (200 Torr) getrocknet, durch ein Sieb mit 1,0 mm Maschenweite egalisiert und abschließend wiederum 3 Minuten gemischt. Das erhaltene Granulat wird zum Gebrauch mit 1 Liter Wasser vermischt und innerhalb 5 Minuten nach Herstellung verwendet.

## Herstellungsbeispiel 2

A) Herstellung der Granulierlösung

Die Lösung von 743 mg (1 mMol) Mono-N-Methylglucaminsalz des Gadolinium-III-Komplexes der Diethylentriaminpentaessigsäure (DTPA), 195,2 mg (1 mMol) N-Methylglucamin und 2,423 g (20 mMol)

Trishydroxymethylaminomethan in 5 ml Wasser wird mit 1n Salzsäure auf den pH-Wert 7,4 bis 7,5 gebracht und auf ein Volumen von 10 ml aufgefüllt.

B) 55 g Mannit wird durch ein Sieb mit 0,8 mm Maschenweite gegeben und mit der nach A) hergestellten Granulierlösung (10 ml) portionsweise befeuchtet und angerieben und, wie im Herstellungsbeispiel 1 unter B) beschrieben, weiter verfahren.

Herstellungsbeispiel 3

A) Die Herstellung der Granulierlösung erfolgt wie im Herstellungsbeispiel 1 unter A) beschrieben.

B) Man mischt 45 g Mannit, das vorher über ein Sieb mit 0,4 mm Maschenweite gegeben wurde und 5 g Traganth ca. 3 Minuten, gibt die Mischung durch ein Sieb mit 0,8 mm Maschenweite und befeuchtet diese Mischung mit der nach A) hergestellten Granulierlösung. Das feuchte Granulat wird, wie im Herstellungsbeispiel 1 unter B) beschrieben, weiter verarbeitet und abschließend über ein sieb mit 0,8 mm Maschenweite gegeben.

**Patentansprüche**

1. Enterales Kontrastmittel für die Protonenkernspintomographie, enthaltend mindestens eine physiologisch verträgliche paramagnetische Verbindung in Kombination mit einem physiologisch verträglichen osmotisch wirksamen Polyol sowie physiologisch verträgliche Base/Puffer oder Puffergemisch mit einem pH-Wert von 3 bis 8 gegebenenfalls zusätzlich einen viskositätserhöhenden Stoff, gelöst oder suspendiert in Wasser.

2. Enterales Kontrastmittel gemäß Anspruch 1, dadurch gekennzeichnet, daß es als physiologisch verträgliche paramagnetische Verbindung Eisen-III-Verbindungen oder Komplexsalze aus Aminopolysäuren und den Ionen der Lanthaniden-Elemente der Ordnungszahlen 57 bis 70 oder den Ionen der Übergangsmetalle der Ordnungszahlen 21 bis 29, 42 und 44 und gegebenenfalls einer anorganischen oder organischen Base enthält.

3. Enterales Kontrastmittel gemäß Anspruch 1, dadurch gekennzeichnet, daß es als physiologisch verträgliches, osmotisch wirksames Polyol Mannit, Sorbit, Arabit, oder Xylit in einer Menge von 5 bis 70 Gramm pro Liter enthält.

4. Enterales Kontrastmittel gemäß Anspruch 1, dadurch gekennzeichnet, daß es als gegebenenfalls zusätzlich vorhandenen viskositätserhöhenden Stoff natürliche oder halbsynthetische hochmolekulare Kohlenhydrate, Polypeptide oder quellfähige Silikate in einer Menge von 2 bis 40 g pro Liter enthält.

5. Enterales Kontrastmittel gemäß Ansprüche 1 und 4, dadurch gekennzeichnet, daß es als viskositätserhöhenden Stoff natürliche hochmolekulare Kohlenhydrate wie Traganth, Bassorin, Guar oder Methylhydroxyethylcellulose enthält.

6. Enterales Kontrastmittel gemäß der Ansprüche 1 und 4, dadurch gekennzeichnet, daß es als viskositätserhöhenden Stoff halbsynthetische hochmolekulare Kohlenhydrate wie Natriumcarboxymethylcellulose oder Methylcellulose und deren Hydroxyalkylderivate wie Methylhydroxyethylcellulose enthält.

7. Enterales Kontrastmittel gemäß Anspruch 1, dadurch gekennzeichnet, daß es als physiologisch verträgliches Komplexsalz das Di-N-Methylglucaminsalz, das Di-Natriumsalz oder das N-Methylglucamin-Natrium-Mischsalz des Gadolinium-III-Komplexes der Diäethylentriaminpentaessigsäure oder das Di-N-Methylglucaminsalz des Eisen-III-Komplexes der Diäthylentriaminpentaessigsäure enthält.

8. Enterales Kontrastmittel gemäß Anspruch 1, dadurch gekennzeichnet, daß es das Di-N-Methylglucaminsalz, das Di-Natriumsalz oder das N-Methylglucamin-Natrium-Mischsalz des Gadolinium-III-Komplexes der Diäthylentriaminpentaessigsäure zusammen mit Mannit und Trishydroxymethylaminomethan (Tromethanol) enthält.

9. Enterales Kontrastmittel gemäß Anspruch 1, dadurch gekennzeichnet, daß es 0,05 bis 5 mMol pro Liter Di-N-Methylglucaminsalz, Di-Natriumsalz oder N-Methylglucamin-Natrium-Mischsalz des Gadolinium-III-Komplexes der Diäthylentriaminpentaessigsäure, 10 bis 50 mMol pro Liter Trishydroxymethylaminomethan und 20 bis 60 g pro Liter Mannit in Wasser enthält.

10. Enterales Kontrastmittel gemäß Anspruch 1, dadurch gekennzeichnet, daß es 0,05 bis 5 mMol pro Liter Di-Methylglucaminsalz, Di-Natriumsalz oder N-Methylglucamin-Natrium-Mischsalz des Gadolinium-III-Komplexes der Diäthylentriaminpentaessigsäure, 10 bis 50 mMol pro Liter Trishydroxymethylaminomethan, 20 bis 60 g pro Liter Mannit und 5 bis 30 g pro Liter Methylhydroxyethylcellulose (Methocel) in Wasser enthält.

11. Enterales Kontrastmittel gemäß Anspruch 1, dadurch gekennzeichnet, daß es 0,05 bis 5 mMol pro Liter Di-N-Methylglucaminsalz, Di-Natriumsalz oder N-Methylglucamin-Natrium Mischsalz des Gadolinium-III-Komplexes der Diethylentriaminpentaessigsäure, 10 bis 50 mMol pro Liter Trishydroxymethylaminomethan, 20 bis 60 g pro Liter Mannit und 2 bis 20 g pro Liter Traganth in Wasser enthält.

12. Verfahren zur Herstellung eines enteralen Kontrastmittels für die Kernspintomographie gemäß

der Ansprüche 1 bis 11, dadurch gekennzeichnet, daß man in an sich bekannter Weise ein Granulat, enthaltend mindestens ein physiologisch verträgliches paramagnetisohes Komplexsalz zusammen mit einer physiologisch verträglichen osmotisch wirksamen Substanz sowie physiologisch verträgliche Base/Puffer oder Puffergemisch mit einem pH-Wert von 3 bis 8 und gegebenenfalls zusätzlich einen physiologisch verträglichen viskositätserhöhenden Stoff, in Wasser löst oder suspendiert.

**Claims**

1. Enteral contrast medium for proton nuclear spin tomography, containing at least one physiologically tolerable, paramagnetic compound in conjunction with a physiologically tolerable, osmotically active polyol, as well as a physiologically tolerable base/buffer or buffer mixture with a pH value of from 3 to 8 and optionally, in addition, a viscosity-increasing substance, dissolved or suspended in water.

2. Enteral contrast medium according to claim 1, characterised in that it contains, as a physiologically tolerable paramagnetic compound, iron(III) compounds or complex salts of aminopolyacids and the ions of the lanthanide elements of atomic numbers 57 to 70 or the ions of the transition metals of atomic numbers 21 to 29, 42 and 44, and optionally an inorganic or organic base.

3. Enteral contrast medium according to claim 1, characterised in that it contains, as a physiologically tolerable, osmotically active polyol, mannitol, sorbitol, arabitol or xylitol in an amount of from 5 to 70 grams per litre.

4. Enteral contrast medium according to claim 1, characterised in that it contains, as an optionally additionally present, viscosity-increasing substance, natural or semisynthetic, high-molecular carbohydrates, polypeptides, or swellable silicates in an amount of from 2 to 40 g per litre.

5. Enteral contrast medium according to claims 1 and 4, characterised in that it contains, as a viscosity-increasing substance, natural, high-molecular carbohydrates, such as tragacanth, bassorin, guar or methylhydroxyethylcellulose.

6. Enteral contrast medium according to claims 1 and 4, characterised in that it contains, as a viscosity-increasing substance, semi-synthetic, high-molecular carbohydrates, such as sodium carboxymethylcellulose or methylcellulose, and the hydroxyalkyl derivatives thereof, such as methylhydroxyethylcellulose.

7. Enteral contrast medium according to claim 1, characterised in that it contains, as a physiologically tolerable complex salt, the di-N-methylglucamine salt, the disodium salt, or the N-methylglucamine-sodium mixed salt of the gadolinium(III) complex of diethylenetriaminepentaacetic acid or the di-N-methylglucamine salt of the iron(III) complex of diethylenetriaminepentaacetic acid.

8. Enteral contrast medium according to claim 1, characterised in that it contains the di-N-methylglucamine salt, the disodium salt, or the N-methylglucamine-sodium mixed salt of the gadolinium(III) complex of diethylenetriaminepentaacetic acid, together with mannitol and trishydroxymethylaminomethane (Tromethanol).

9. Enteral contrast medium according to claim 1, characterised in that it contains from 0.05 to 5 mmols per litre of di-N-methylglucamine salt, disodium salt, or N-methylglucamine-sodium mixed salt of the gadolinium(III) complex of diethylenetriaminepentaacetic acid, from 10 to 50 mmols per litre of trishydroxymethylaminomethane, and from 20 to 60 g per litre of mannitol in water.

10. Enteral contrast medium according to claim 1, characterised in that it contains from 0.05 to 5 mmols per litre of di-methylglucamine salt, disodium salt, or N-methylglucamine-sodium mixed salt of the gadolinium(III) complex of diethylenetriaminepentaacetic acid, from 10 to 50 mmols per litre of trishydroxymethylaminomethane, from 20 to 60 g per litre of mannitol, and from 5 to 30 g per litre of methylhydroxyethylcellulose (Methocel) in water.

11. Enteral contrast medium according to claim 1, characterised in that it contains from 0.05 to 5 mmols per litre of di-N-methylglucamine salt, disodium salt, or N-methylglucamine-sodium mixed salt of the gadolinium(III) complex of diethylenetriaminepentaacetic acid, from 10 to 50 mmols per litre of trishydroxymethylaminomethane, from 20 to 60 g per litre of mannitol, and from 2 to 20 g per litre of tragacanth in water.

12. Process for the production of an enteral contrast medium for nuclear spin tomography according to claims 1 to 11, characterised in that, in a manner known per se, a granulate containing at least one physiologically tolerable paramagnetic complex salt is dissolved or suspended in water together with a physiologically tolerable, osmotically active substance, as well as a physiologically tolerable base/buffer or buffer mixture with a pH value of from 3 to 8 and optionally, in addition, a physiologically tolerable, viscosity-increasing substance.

**Revendications**

1. Agent de contraste entéral pour la tomographie par spin nucléaire protonique, agent qui contient au moins un composé paramagnétique acceptable du point de vue physiologique, associé à un polyol à action osmotique, acceptable du point de vue physiologique, ainsi qu'à une base ou un tampon ou un

mélange tampon acceptable du point de vue physiologique et dont le pH est de 3 à 8, et éventuellement, en plus, à une substance augmentant la viscosité, en solution ou en suspension dans de l'eau.

2. Agent de contraste entéral selon la revendication 1, caractérisé en ce qu'il contient, comme composé paramagnétique acceptable du point de vue physiologique, un composé du fer(III) ou un sel complexe d'un amino-polyacide et d'un ion d'un élément pris dans l'ensemble constitué par les lanthanides de numéros atomiques allant de 57 à 70 et les métaux de transition de numéros atomiques allant de 21 à 29, 42 et 44, et, éventuellement, d'une base minérale ou organique.

3. Agent de contraste entéral selon la revendication 1, caractérisé en ce qu'il contient, comme polyol à activité osmotique, acceptable du point de vue physiologique, du mannitol, du sorbitol, de l'arabitol ou du xylitol en une quantité de 5 à 70 g par litre.

4. Agent de contraste entéral selon la revendication 1, caractérisé en ce qu'il contient, comme substance augmentant la viscosité, qui est éventuellement présente en plus, un glucide macromoléculaire naturel ou semisynthétique, un polypeptide ou un silicate gonflable, en une quantité de 2 à 40 g par litre.

5. Agent de contraste entéral selon l'une des revendications 1 et 4, caractérisé en ce qu'il contient, comme substance augmentant la viscosité, un glucide macromoléculaire naturel, tel que l'adragante, la bassorine, le guar ou la méthyl-hydroxyéthyl-cellulose.

6. Agent de contraste entéral selon l'une des revendications 1 et 4, caractérisé en ce qu'il contient, comme substance augmentant la viscosité, un glucide macromoléculaire semisynthétique, tel que le dérivé sodique de la carboxyméthyl-cellulose ou la méthyl-cellulose ou ses dérivés hydroxyalkylés tels que la méthyl-hydroxyéthyl-cellulose.

7. Agent de contraste entéral selon la revendication 1, caractérisé en ce qu'il contient, comme sel complexe acceptable du point de vue physiologique, le sel de di-(N-méthyl-glucamine), le sel disodique ou le sel mixte de N-méthyl-glucamine et de sodium du complexe de gadolinium(III) de l'acide diéthylène-triamine-pentacétique, ou le sel de di-(N-méthyl-glucamine) du complexe de fer(III) de l'acide diéthylène-triamine-pentacétique.

8. Agent de contraste entéral selon la revendication 1, caractérisé en ce qu'il contient le sel de di-(N-méthyl-glucamine), le sel disodique ou le sel mixte de N-méthyl-glucamine et de sodium du complexe de gadolinium(III) de l'acide diéthylène-triamine-pentacétique, associé au mannitol et au tris-(hydroxyméthyl)-amino-méthane (ou trométamol).

9. Agent de contraste entéral selon la revendication 1, caractérisé en ce qu'il contient, par litre, de 0,05 à 5 mmol du sel de di-(N-méthyl-glucamine), du sel disodique ou du sel mixte de N-méthyl-glucamine et de sodium du complexe de gadolinium(III) de l'acide diéthylène-triamine-pentacétique, de 10 à 50 mmol de tris-(hydroxyméthyl)-amino-méthane et de 20 à 60 g de mannitol dans de l'eau.

10. Agent de contraste entéral selon la revendication 1 caractérisé en ce qu'il contient, par litre, de 0,05 à 5 mmol du sel de di-(N-méthyl-glucamine), du sel disodique ou du sel mixte de N-méthyl-glucamine et de sodium du complexe de gadolinium(III) de l'acide diéthylène-triamine-pentacétique, de 10 à 50 mmol de tris-(hydroxyméthyl)-amino-méthane, de 20 à 60 g de mannitol et de 5 à 30 g de méthyl-hydroxyéthyl-cellulose (méthocel) dans de l'eau.

11. Agent de contraste entéral selon la revendication 1, caractérisé en ce qu'il contient, par litre, de 0,05 à 5 mmol du sel de di-(N-méthyl-glucamine), du sel disodique ou du sel mixte de N-méthyl-glucamine et de sodium du complexe de gadolinium(III) de l'acide diéthylène-triamine-pentacétique, de 10 à 50 mmol de tris-(hydroxyméthyl)-amino-méthane, de 20 à 60 g de mannitol et de 2 à 20 g d'adragante dans de l'eau.

12. Procédé pour préparer un agent de contraste entéral pour la tomographie par spin nucléaire selon l'une quelconque des revendications 1 à 11, procédé caractérisé en ce que, en opérant de manière connue, on dissout ou met en suspension dans de l'eau un produit granulé qui contient au moins un sel complexe paramagnétique, acceptable du point de vue physiologique, une substance à activité osmotique, acceptable du point de vue physiologique, ainsi qu'une base ou un tampon, ou un mélange tampon, acceptable du point de vue physiologique, dont le pH est de 3 à 8, et éventuellement, en plus, une substance augmentant la viscosité, acceptable du point de vue physiologique.